# EUROPEAN PATENT APPLICATION

(11) **EP 4 679 090 A1**
(43) Date of publication of application: **14.01.2026**
(21) Application number: 24187510.3
(22) Date of filing: 09.07.2024
(51) Int. Cl.: G01N 33/542, C12N 9/00

(54) **A TARGET-DIRECTED AND CRL-AGNOSTIC PROXIMITY ASSAY**

(71) Applicant: Proxygen GmbH, 1210 Vienna (AT)
(72) Inventor: The designation of the inventor has not yet been filed
(74) Representative: Maiwald GmbH

(57) **Abstract**

The present invention relates to *in vitro* methods of (a) determining whether a compound induces proximity between (i) a target protein and (ii) a Cullin-RING E3 ubiquitin ligase complex; (b) of screening a plurality of different compounds for the ability of a compound of said plurality of different compounds to induce proximity between (i) and (ii); (c) confirming that a compound induces proximity between (i) and (ii); and (d) of improving the ability of a compound to induce proximity between (i) and (ii) to arrive at an improved compound.

## Description

### TECHNICAL FIELD

The present invention is in the field of targeted protein degradation. It is concerned with (a) an *in vitro* method of determining whether a compound induces proximity between (i) a target protein and (ii) a Cullin-RING E3 ubiquitin ligase complex; (b) an *in vitro* method of screening a plurality of different compounds for the ability of a compound of said plurality of different compounds to induce proximity between (i) a target protein and (ii) a Cullin-RING E3 ubiquitin ligase complex; (c) an *in vitro* method of confirming that a compound induces proximity between (i) a target protein and (ii) a Cullin-RING E3 ubiquitin ligase complex; and (d) an *in vitro* method of improving the ability of a compound to induce proximity between (i) a target protein and (ii) a Cullin-RING E3 ubiquitin ligase complex to arrive at an improved compound.

### BACKGROUND OF THE INVENTION

Proximity and molecular recognition are two fundamental mechanisms for relaying information within and between cells, as mentioned in the introductory section of Liu and Ciulli, ACS Cent. Sci. 2023, 9, 1269-1284. As also mentioned in the introductory section therein, it was not until the 1990s that artificially inducing proximity was realized to be sufficient to initiate signaling events, when it was found that homodimerizing T cell receptors (TCRs) using antibodies or synthetic dimerizer FK1012 could recapitulate TCR signaling in the absence of a T-lymphocyte antigen. Similar approaches were later applied to active Ras signaling, death receptor signaling, and transcription, as also disclosed in Liu and Ciulli, *supra.*

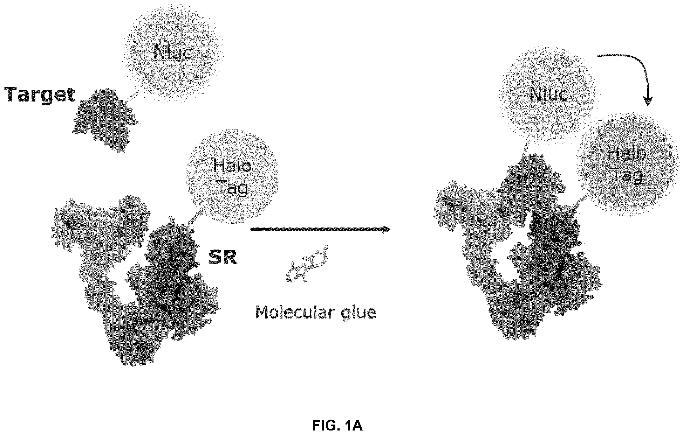

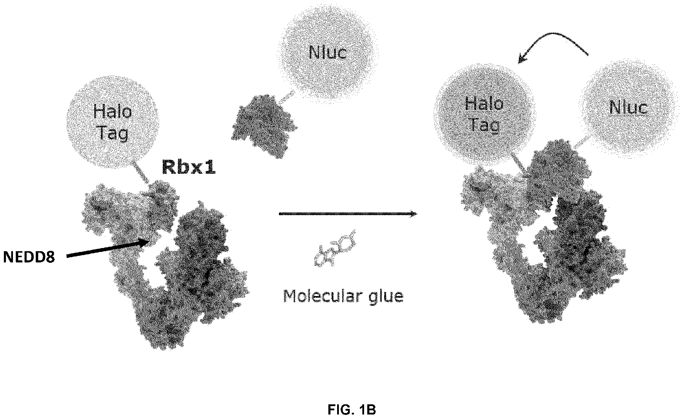

Compared to these rather early findings, there is a more recent field that also relies on proximity, namely the field of targeted protein degradation, where proximity is induced chemically between a target protein to be degraded and a ubiquitin E3 ligase. The goal is that this proximity triggers ubiquitination of the target protein and accordingly subsequent degradation of the target protein by the proteasome. Particular target proteins of interest are proteins implicated in diseases, which have proven to be difficult targets for the more classical approaches of modulating their activity (e.g. proteins lacking catalytic sites or more generally devoid of pockets on their surface amenable to small-molecule binding). When it comes to ubiquitin E3 ligases, a particular focus is on Cullin-RING (really interesting new gene) E3 ubiquitin ligase (CRL) complexes because they are the largest family of E3 ubiquitin ligases. As shown e.g. in Fig. 1A of Bulatov and Ciulli, Biochem. J. (2015) 467, 365-386, CRL complexes have a general subunit organization of substrate receptor, adaptor, Cullin scaffold and RING-protein subunits. Examples of Cul1 to Cul7 complexes are depicted in Table 1 of Bulatov and Ciulli, *supra,* with e.g. the Cul1 complex comprised of the substrate receptor subunit being an F-box protein (such as Skp2), the adaptor Skp1 and the RING protein RBX1 or the Cul4 complex comprised of the substrate receptor subunit being a DCAF/H-box protein (such as DCAF), the adaptor DBB1 and the RING protein RBX1. A schematic view of CRL complexes including the substrate and the (ubiquitin-charged) E2 enzymes can also be found for the Cul1 (or CRL1) to Cul5 (or CLR5) complexes in Fig. 1 of Zimmerman et al., Current Opinion in Structural Biology 2010, 20:714-721. Furthermore, CRL complexes are typically regulated in that the activity is altered and/or the association/dissociation cycles of CRL subunits are modulated, wherein NEDD8 is such a regulatory protein that is present in all CRL complexes.

Chemically induced proximity between a target protein and a ubiquitin ligase can in particular be triggered by two classes of compounds, namely proteolysis-targeting chimeras (PROTACs) and molecular glue degraders. While molecular glue degraders do not have a modular setup, PROTACs are heterobifunctional molecules consisting of two binding modules that are connected by a linker: one binding module binds to the target protein and one binding module binds to an E3 ligase component, wherein the above mentioned substrate receptor of CRL complexes is typically the E3 ligase component that is targeted, with binding modules binding to the substrate receptors VHL and CRBN being best characterized and most often used.

The above mentioned chemically induced proximity between a target protein and a ubiquitin ligase may also be referred to as ternary complex formation, and there are methods to assess such a ternary complex formation or induced proximity, respectively. Thus, as shown e.g. in Table I in Daniels et al., Drug Discovery Today: Technologies /Protein degradation for drug discovery, 2019, Vol. 31, 61-68, an approach to assess ternary complex formation on a high throughput level is the nanoBRET protein:protein interaction (PPI) assay. The assay as such is described in more detail in the section bridging pages 64 and 65 of Daniels et al., *supra,* wherein it is highlighted that small changes to the linker of a PROTAC can have a significant impact on ternary complex formation, while also electrostatic surface interactions created by the PROTAC between the target protein and the E3 ligase component are of relevance for the stability of the ternary complex. Overall, the assay provides valuable information as regards the induction of proximity, which is a necessary precursor for facilitating downstream ubiquitination, and in guiding structure-activity relationship (SAR) development of compounds for improving degradation. Daniels et al., *supra,* mention two examples of structural studies that have shed light into the phenomen of electrostatic surface interactions, wherein both include as target protein BRD4, while the substrate receptor is in the first example VHL (with MZ1 being the PROTAC) and in the second example CRBN (with the PROTAC being dBET23). Thus, these examples are in line with the above reference to the substrate receptors VHL and CRBN as being the best characterized and most often used components of CRL complexes, to which the binding modules bind to. While other PROTACs and therefore also the assays assessing such PROTACs may rely on different substrate receptors, it is common to the methods used today to assess the induction of proximity by tagging both the target protein and a substrate receptor of a CRL complex.

A drawback of the above method is that the induction of proximity is exclusively analyzed for a specific CRL complex, namely for the CRL complex comprising the substrate receptor that is tagged (e.g. a Cul2 complex if VHL is tagged or a Cul4A complex if CRBN is tagged). Due to the modular nature of PROTACs, typically it is known which is the relevant CRL complex. If the NanoBRET PPI assay is, however, carried out with a potential molecular glue or a library of potential molecular glues, respectively, it is completely unclear between which specific CRL complex out of the plurality of CRL complexes and the target protein proximity is chemically induced. As consequence, the NanoBRET PPI assay needs to be carried out in a parallel format using an identical tagged target protein in all assays but different tagged substrate receptors in parallel assays in order not to miss the specific CRL complex that is brought into proximity to the target protein by a molecular glue. Due to the high number of possible CRL complexes, with more than 200 different substrate receptor subunits reported to be associated with CRLs (see Petroski and Deshaies, Nat Rev Mol Cell Biol. 2005 Jan; 6(1):9-20), a systematic assessment of all possible CRL complexes is in reality not viable.

In view of the above, there is the need for a method to assess whether a compound induces proximity between a target protein and a CRL complex in general, i.e. irrespective of the specific substrate receptor or ideally irrespective of the specific CRL complex. In other words, there is the need for a method to assess whether a compound induces proximity between a target protein and a CRL complex, wherein the method is agnostic towards the substrate receptor and ideally towards the CRL complex.

### SUMMARY OF THE INVENTION

The present invention solves the above need in that a method is provided, in which RBX1 or NEDD8 is fused to a tag. In contrast to the substrate receptor subunit of CRL complexes, which is specific for each of the different CRL complexes, RBX1 is a RING protein subunit that is present in substantially all CRL complexes and NEDD8 is a regulatory protein that is present in substantially all active CRL complexes such that the method of the present invention is capable of determining whether a compound induces proximity between a target protein and a CRL complex in the meaning of any CRL complex or in the meaning that the method is agnostic towards the substrate receptor and the CRL complex, respectively.

In a **first aspect,** the present application is directed to an *in vitro* method of determining whether a compound induces proximity between (i) a target protein and (ii) a Cullin-RING E3 ubiquitin ligase (CRL) complex, the method comprising the following steps:
a. Providing a cell comprising
   i. a target protein fused to a protein being an energy donor and RBX1 or NEDD8 fused to a protein being an energy acceptor; or
   ii. a target protein fused to a protein being an energy acceptor and RBX1 or NEDD8 fused to a protein being an energy donor; or
   iii. a target protein fused to a protein being a first protein fragment of a signal-generating protein and RBX1 or NEDD8 fused to a protein being a second fragment of the signal-generating protein, wherein the first fragment and the second fragment are, upon association, capable of forming a functional signal-generating protein;
b. Adding a compound to the cell provided in step a); and
c. Determining for a)i) and a)ii) whether there is energy transfer from the energy donor to the energy acceptor and for a)iii) whether there is a signal of the signal-generating protein in the cell to which the compound has been added in step b);
wherein for a)i) and a)ii) the presence of energy transfer and for a)iii) the presence of a signal indicates that the compound induces proximity between (i) and (ii).

The *in vitro* method of the first aspect may alternatively be referred to as *in vitro* method of determining whether a compound induces complex formation between (i) and (ii), wherein the method comprises the above steps and wherein for a)i) and a)ii) the presence of energy transfer and for a)iii) the presence of a signal indicates that the compound induces complex formation between (i) and (ii).

Yet alternatively, the *in vitro* method of the first aspect may be referred to as *in vitro* method of determining whether a compound induces a complex comprising (i) and (ii), wherein the method comprises the above steps and wherein for a)i) and a)ii) the presence of energy transfer and for a)iii) the presence of a signal indicates that the compound induces a complex comprising (i) and (ii).

Yet alternatively, the *in vitro* method of the first aspect may be referred to as *in vitro* method of determining whether a compound induces recruitment of (i) to (ii), wherein the method comprises the above steps and wherein for a)i) and a)ii) the presence of energy transfer and for a)iii) the presence of a signal indicates that the compound induces recruitment of (i) to (ii).

Yet alternatively, the *in vitro* method of the first aspect may be referred to as *in vitro* method of determining complex formation between (i) and (ii), wherein the method comprises the above steps and wherein for a)i) and a)ii) the presence of energy transfer and for a)iii) the presence of a signal indicates complex formation between (i) to (ii).

In a **second aspect,** the present invention is directed to an *in vitro* method of screening a plurality of different compounds for the ability of a compound of said plurality of different compounds to induce proximity between (i) a target protein and (ii) a Cullin-RING E3 ubiquitin ligase complex, the method comprising the following steps:
a. Providing a plurality of cells, each cell comprising
   i. a target protein fused to a protein being an energy donor and RBX1 or NEDD8 fused to a protein being an energy acceptor; or
   ii. a target protein fused to a protein being an energy acceptor and RBX1 or NEDD8 fused to a protein being an energy donor; or
   iii. a target protein fused to a protein being a first protein fragment of a signal-generating protein and RBX1 or NEDD8 fused to a protein being a second fragment of the signal-generating protein, wherein the first fragment and the second fragment are, upon association, capable of forming a functional signal-generating protein;
b. Adding each compound of said plurality of different compounds to at least one given cell of the plurality of cells provided in step a) and thereby providing a plurality of given cells, wherein each at least one given cell comprises a specific compound of said plurality of different compounds; and
c. Determining in which of the at least one given cell provided in step b) there is for a)i) and a)ii) energy transfer from the energy donor to the energy acceptor and for a)iii) a signal of the signal-generating protein;
wherein the presence of energy transfer for a)i) and a)ii) and the presence of a signal for a)iii) in an at least one given cell indicates that the specific compound of said plurality of compounds comprised therein has the ability to induce proximity between (i) and (ii).

The *in vitro* method of the second aspect may alternatively be referred to as *in vitro* method of screening a plurality of different compounds for the ability of a compound of said plurality of different compounds to induce complex formation between (i) and (ii), wherein the method comprises the above steps and wherein for a)i) and a)ii) the presence of energy transfer and for a)iii) the presence of a signal indicates that the compound induces complex formation between (i) and (ii).

Yet alternatively, the *in vitro* method of the second aspect may be referred to as *in vitro* method of screening a plurality of different compounds for the ability of a compound of said plurality of different compounds to induce a complex comprising (i) and (ii), wherein the method comprises the above steps and wherein for a)i) and a)ii) the presence of energy transfer and for a)iii) the presence of a signal indicates that the compound induces a complex comprising (i) and (ii).

Yet alternatively, the *in vitro* method of the second aspect may be referred to as *in vitro* method of determining whether a compound induces recruitment of (i) to (ii), wherein the method comprises the above steps and wherein for a)i) and a)ii) the presence of energy transfer and for a)iii) the presence of a signal indicates that the compound induces recruitment of (i) to (ii).

In a **third aspect,** the present invention is directed to an *in vitro* method of confirming that a compound induces proximity between (i) a target protein and (ii) a Cullin-RING E3 ubiquitin ligase complex, the method comprising the following steps:
a. Providing a cell comprising
   i. a target protein fused to a protein being an energy donor and RBX1 or NEDD8 fused to a protein being an energy acceptor; or
   ii. a target protein fused to a protein being an energy acceptor and RBX1 or NEDD8 fused to a protein being an energy donor; or
   iii. a target protein fused to a protein being a first protein fragment of a signal-generating protein and RBX1 or NEDD8 fused to a protein being a second fragment of the signal-generating protein, wherein the first fragment and the second fragment are, upon association, capable of forming a functional signal-generating protein;
b. Adding a compound to the cell provided in step a); and
c. Determining for a)i) and a)ii) whether there is energy transfer from the energy donor to the energy acceptor and for a)iii) whether there is a signal of the signal-generating protein in the cell to which the compound has been added in step b);
wherein for a)i) and a)ii) the presence of energy transfer and for a)iii) the presence of a signal confirms that the compound induces proximity between (i) and (ii).

The *in vitro* method of the third aspect may alternatively be referred to as *in vitro* method of confirming that a compound induces complex formation between (i) and (ii), wherein the method comprises the above steps and wherein for a)i) and a)ii) the presence of energy transfer and for a)iii) the presence of a signal confirms that the compound induces complex formation between (i) and (ii).

Yet alternatively, the *in vitro* method of the third aspect may be referred to as *in vitro* method of confirming that a compound induces a complex comprising (i) and (ii), wherein the method comprises the above steps and wherein for a)i) and a)ii) the presence of energy transfer and for a)iii) the presence of a signal confirms that the compound induces a complex comprising (i) and (ii).

Yet alternatively, the *in vitro* method of the third aspect may be referred to as *in vitro* method of confirming that a compound induces recruitment of (i) to (ii), wherein the method comprises the above steps and wherein for a)i) and a)ii) the presence of energy transfer and for a)iii) the presence of a signal indicates that the compound confirms recruitment of (i) to (ii).

In a **fourth aspect,** the present invention is directed to an *in vitro* method of improving the ability of a compound to induce proximity between (i) a target protein and (ii) a Cullin-RING E3 ubiquitin ligase complex to arrive at an improved compound, the method comprising the following steps:
a. Providing a cell comprising
   i. a target protein fused to a protein being an energy donor and RBX1 or NEDD8 fused to a protein being an energy acceptor; or
   ii. a target protein fused to a protein being an energy acceptor and RBX1 or NEDD8 fused to a protein being an energy donor; or
   iii. a target protein fused to a protein being a first protein fragment of a signal-generating protein and RBX1 or NEDD8 fused to a protein being a second fragment of the signal-generating protein, wherein the first fragment and the second fragment are, upon association, capable of forming a functional signal-generating protein;
b. Adding a compound to the cell provided in step a);
c. Quantifying for a)i) and a)ii) the energy transfer from the energy donor to the energy acceptor and for a)iii) the signal of the signal-generating protein in the cell to which the compound has been added in step b) to arrive at result 1;
d. Modifying the compound added in step b) to arrive at a modified compound;
e. Adding the modified compound of step d) to the cell provided in step a); and
f. Quantifying for a)i) and a)ii) the energy transfer from the energy donor to the energy acceptor and for a)iii) the signal of the signal-generating protein in the cell to which the modified compound has been added in step e) to arrive at result 2;
wherein a ratio of result 2 / result 1 of >1 indicates that the modified compound of step d) is an improved compound.

The *in vitro* method of the fourth aspect may alternatively be referred to as *in vitro* method of improving the ability of a compound to induce complex formation between (i) and (ii), wherein the method comprises the above steps.

Yet alternatively, the *in vitro* method of the fourth aspect may be referred to as *in vitro* method of improving the ability of a compound to induce a complex comprising (i) and (ii), wherein the method comprises the above steps.

Yet alternatively, the *in vitro* method of the fourth aspect may be referred to as *in vitro* method of improving the ability of a compound to induce recruitment of (i) to (ii), wherein the method comprises the above steps.

In an embodiment of the fourth aspect, the compound added in step b) and the modified compound added in step e) are added at the same concentration.

In an embodiment of any of the above aspects, the compound is capable of crossing a cell membrane in order to be present inside a cell. The crossing of the cell membrane may be active (i.e. an active internalization, in particular via a receptor) or passive (i.e. a passive internalization, in particular via diffusion through the lipid bilayer of the cell membrane).

In an embodiment of any of the above aspects, the compound is selected from the group consisting of a small molecule, a PROTAC, a peptide, a nucleic acid (RNA or DNA) fused to a small molecule or a peptide, an aptamer, a fused antibody, and a macrocycle. In a preferred embodiment of any of the above aspects, the compound is a PROTAC, a small molecule, or a peptide. In a preferred embodiment of any of the above aspects, the compound is not a PROTAC. In a preferred embodiment of any of the above aspects, the compound is a small molecule. If the compound is a small molecule, the small molecule can in particular be a (potential) molecular glue.

In an embodiment of any of the above aspects, the target protein is a protein that is overexpressed and/or overactivated in a cell of a human resulting in a disease of the human, such as cancer. The target protein may be a protein not comprising a hydrophobic binding pocket and/or a binding site such that this target protein can be characterized as being "undruggable" by classical modulatory compounds. Examples of "undruggable" target proteins are e.g. small GTPases, phosphatases or transcription factors.

In an embodiment of any of the above aspects, the Cullin-RING E3 ubiquitin ligase complex is a Cullin-RING E3 ubiquitin ligase complex comprising RBX1 and NEDD8. It can be preferred that the Cullin-RING E3 ubiquitin ligase complex is selected from the group consisting of the Cul1 complex, the Cul2 complex, the Cul3 complex, the Cul4A complex, the Cul4B complex, the Cul5 complex and the Cul7 complex.

In an embodiment of any of the above aspects, the cell is a eukaryotic cell. In a preferred embodiment of any of the above aspects, the eukaryotic cell is a human cell or derived from a human cell line, respectively. It is preferred that the cell is a human cell or derived from a human cell line, respectively, and selected from the group consisting of a HEK293 cell, a HEK293T cell, a RKO cell, an A549 cell, a DLD-1 cell, a KBM7 cell, a HeLa cell, and an HCT116 cell.

In a preferred embodiment of any of the above aspects, step a. comprises i) a target protein fused to a protein being an energy donor and RBX1 or NEDD8 fused to a protein being an energy acceptor.

In an embodiment of any of the above aspects, the protein fused to the target protein is fused N-terminally or C-terminally to the target protein.

In an embodiment of any of the above aspects, the protein fused to RBX1 or NEDD8 is fused N-terminally or C-terminally to RBX1 or NEDD8. In a preferred embodiment of any of the above aspect, the protein fused to RBX1 or NEDD8 is fused N-terminally to RBX1 or NEDD8.

In an embodiment of any of the above aspects, a linker is present between the target protein or RBX1/NEDD8, respectively, and the protein fused thereto. Thus, if the fusion is N-terminally, the resulting protein may have the setup of protein-linker-target protein and protein-linker-RBX1/NEDD8, respectively. If the fusion is C-terminally, the resulting protein may have the setup of target protein-linker-protein and RBX1/NEDD8-linker-protein, respectively. It is preferred that the linker consists of a peptide, wherein the peptide may consist of about 2 to about 26 amino acids, preferably the amino acids G and S. It can be particularly preferred that the linker has the amino acid sequence of SEQ ID NO:13 or SEQ ID NO:14 or SEQ ID NO:25 or SEQ ID NO:26 or GGGGSGGGGS (SEQ ID NO:28). Alternatively, it can be preferred that the linker has the amino acid sequence of EAAAK (SEQ ID NO:29) or (EAAAK)n with n being typically about 2 to about 4, specifically EAAAKEAAAK (SEQ ID NO:30) or EAAAKEAAAKEAAAK (SEQ ID NO:31).

In an embodiment of any of the above aspects, a peptide tag is additionally present either at the very N-terminus or at the very C-terminus of the fusion protein. Such a peptide tag can e.g. serve to pull-down the complex comprising the so-tagged protein. A suitable peptide tag can in particular be a HA-tag or a 2xHA-tag (for example the 2xHA-tag with the amino acid sequence of SEQ ID NO:18); a FLAG-tag with the sequence of DYKDDDK (SEQ ID NO:32) or (DYKDDDK)n with n being typically 1 to about 4, specifically DYKDDDKDYKDDDK (SEQ ID NO:33) or DYKDDDKDYKDDDKDYKDDDK (SEQ ID NO:34); and a V5-tag with the sequence GKPIPNPLLGLDST (SEQ ID NO:35) or GKPIPNPLLGLDSTGKPIPNPLLGLDST (SEQ ID NO:36).

In a preferred embodiment of any of the above aspects, the protein being an energy donor is a luciferase. It can be particularly preferred that the luciferase is a nanoluciferase. A suitable nanoluciferase is e.g. the nanoluciferase with the amino acid sequence of SEQ ID NO:16. In such an embodiment, it can be particularly preferred that the protein being an energy acceptor is an enzyme, wherein the enzyme is HaloTag. A suitable HaloTag is e.g. the HaloTag with the amino acid sequence of SEQ ID NO:15. In an embodiment of any of the above aspects, the protein being an energy acceptor is an enzyme bound to a ligand being a fluorophore. It can be preferred in this embodiment that the ligand is added to the cell and reacts with the enzyme to form an enzyme bound to the ligand, preferably an enzyme covalently bound to the ligand. A suitable ligand can e.g. be a HaloTag Ligand, in particular the HaloTag 618 Ligand. In a preferred embodiment any of the above aspects, the protein being an energy acceptor is HaloTag bound to a HaloTag Ligand (in particular to HaloTag 618 Ligand), wherein the binding is preferably covalently.

In a preferred embodiment of any of the above aspect, the protein being an energy donor is a fluorescent protein or a fluorescent ligand; and the protein being an energy acceptor is a fluorescent protein (which is of course different from the afore-mentioned fluorescent protein) or a fluorescent ligand (which is of course different from the afore-mentioned fluorescent ligand).

In a preferred embodiment of any of the above aspects, the first protein fragment of a signal-generating protein is a fragment of a luciferase; and the second protein fragment of a signal-generating protein is a fragment of a luciferase (which is of course different from the fragment mentioned above as first fragment), wherein it can be preferred that the luciferase is a nanoluciferase. The first protein fragment can in particular have the amino acid sequence of either VSGWRLFKKIS (SEQ ID NO:37) or VTGYRLFEEIL (SEQ ID NO:38) ; and the second protein fragment can in particular have the amino acid sequence of

MVFTLEDFVGDWEQTAAYNLDQVLEQGGVSSLLQNLAVSVTPIQRIVRSGENALKIDIHVIIPYEGLS
ADQMAQIEEVFKVVYPVDDHHFKVILPYGTLVIDGVTPNMLNYFGRPYEGIAVFDGKKITVTGTLWN
GNKIIDERLITPDGSMLFRVTINS (SEQ ID NO:39).

In another preferred embodiment of any of the above aspects, the first protein fragment of a signal-generating protein is a fragment of a fluorescent protein; and the second fragment of a signal-generating protein is a fragment of a fluorescent protein (which is of course different from the fragment mentioned above as first fragment), wherein it can be preferred that the fluorescent protein is GFP or a Cherry-protein. The first protein fragment can in particular have the amino acid sequence of MSKGEELFTGVVPILVELDGDVNGHKFSVRGEGEGDATIGKLTLKFICTTGKLPVPWPTLVTTLTYG VQCFSRYPDHMKRHDFFKSAMPEGYVQERTISFKDDGKYKTRAVVKFEGDTLVNRIELKGTDFKED GNILGHKLEYNFNSHNVYITADKQKNGIKANFTVRHNVEDGSVQLADHYQQNTPIGDGPVLLPDNHY LSTQTVLSKDPNEK (SEQ ID NO:40), and the second protein fragment can in particular have the amino acid sequence of RDHMVLHEYVNAAGITGGSGGRDHMVLHEYVNAAGITGGSGGRDHMVLHEYVNAAGITGGSGGR DHMVLHEYVNAAGITGGSGGRDHMVLHEYVNAAGITGGSGGRDHMVLHEYVNAAGITGGSGGRD HMVLHEYVNAAGIT (SEQ ID NO:41). Alternatively, The first protein fragment can in particular have the amino acid sequence of MEEDNMAIIKEFMRFKVHMEGSVNGHEFEIEGEGEGHPYEGTQTARLKVTKGDPLPFAWDILSPQF MYGSKA YVKH PADI PDYLKLSFPEGFTWERVMN FEDGGW A VTQDSSLQDGQFIYKVKLLGI N FPS DGPVMQKKTMGWEASTERMYPEDGALKGEINQRLKLKDGGHYDAEVKTTYRAKKPVQLPGAYDV DIKLDITSHNED (SEQ ID NO:42), and the second protein fragment can in particular have the amino acid sequence of YTIVEQYERAEARHST (SEQ ID NO:43).

In an embodiment of any of the above aspects, the cell comprising the fusion proteins may be obtained by transfecting the cell with at least one nucleic acid construct encoding one or both fusion proteins such that the fusion proteins are either transiently and/or stably expressed in the cell. Suitable methods are exemplified herein in the example section and the skilled person is generally aware of methods in order to provide a cell comprising the fusion proteins.

In an embodiment of any of the above aspects, the compound may be added to the cell by pipetting. In an alternative embodiment of any of the above aspects, the compound may be added to the cell in that the compound is present in a well, into which the cell is provided.

In an embodiment of the first aspect, the second aspect and the third aspect, the determination of energy transfer for a)i) and a)ii) is carried out after adding a substrate of the protein being an energy donor. Such a substrate can in particular be a substrate for a luciferase, preferably a nanoluciferase, e.g. Nano-Glo substrate. In an alternative embodiment of the first aspect, the second aspect and the third aspect, the determination of energy transfer for a)i) and a)ii) is carried out without adding a substrate of the protein being an energy donor but by exciting the protein being an energy donor (which is in this case preferably a fluorescent protein or fluorescent ligand) with light at the suitable wavelength. The determination of energy transfer is typically carried out by measuring emitted light at the suitable donor emission wavelength (e.g. at 460 nm) and detecting light at the suitable acceptor emission wavelength (e.g. at 647 nm), wherein a ratio of acceptor emission value / donor emission value > 1 (normalized against a vehicle-treated control) is indicative of energy transfer. Suitable devices are in particular (micro)plate readers known to the skilled person, with a suitable microplate reader being e.g. the microplate reader used in the present examples.

In an embodiment of the first aspect, the second aspect and the third aspect, the determination of a signal of the signal-generating protein for a)iii) is carried out after adding a substrate of the signal-generating protein. Such a substrate can in particular be a substrate for a luciferase, preferably a nanoluciferase, e.g. Nano-Glo substrate. In an alternative embodiment of the first aspect, the second aspect and the third aspect, the determination of a signal of the signal-generating protein for a)iii) is carried out without adding a substrate of the signal-generating protein but by exciting the protein (which is in this case preferably a fluorescent protein or fluorescent ligand) with light at the suitable wavelength. The determination of a signal is typically carried out by measuring emitted light at the suitable emission wavelength of the signal-generating protein (e.g. at 460 nm), wherein an increase of signal-generating protein emission value above vehicle-treated control is indicative of signal. Suitable devices are in particular (micro)plate readers known to the skilled person, with a suitable microplate reader being e.g. the microplate reader used in the present examples.

In an embodiment of the fourth aspect, the quantification of energy transfer in steps c) and f) for a)i) and a)ii) is carried out after adding a substrate of the protein being an energy donor. Such a substrate can in particular be a substrate for a luciferase, preferably a nanoluciferase, e.g. Nano-Glo substrate. In an alternative embodiment of the fourth aspect, the quantification of energy transfer in steps c) and f) for a)i) and a)ii) is carried out without adding a substrate of the protein being an energy donor but by exciting the protein being an energy donor (which is in this case preferably a fluorescent protein or fluorescent ligand) with light at the suitable wavelength. The quantification of energy transfer is typically carried out by measuring emitted light at the suitable donor emission wavelength (e.g. at 460 nm) and detecting light at the suitable acceptor emission wavelength (e.g. at 647 nm), wherein a ratio of acceptor emission value / donor emission value > 1 (normalized against a vehicle-treated control) provides a result. Suitable devices are in particular (micro)plate readers known to the skilled person, with a suitable microplate reader being e.g. the microplate reader used in the present examples.

In an embodiment of the fourth aspect, the quantification of signal of the signal-generating protein in steps c) and f) for a)iii) is carried out after adding a substrate of the signal-generating protein. Such a substrate can in particular be a substrate for a luciferase, preferably a nanoluciferase, e.g. Nano-Glo substrate. In an alternative embodiment of the fourth aspect, the quantification of signal of the signal-generating protein in steps c) and f) for a)iii) is carried out without adding a substrate of the signal-generating protein but by exciting the protein (which is in this case preferably a fluorescent protein or fluorescent ligand) with light at the suitable wavelength. The quantification of a signal is typically carried out by measuring emitted light at the suitable emission wavelength of the signal-generating protein (e.g. at 460 nm), wherein an increase of signal-generating protein emission value above vehicle-treated control is indicative of signal. Suitable devices are in particular (micro)plate readers known to the skilled person, with a suitable microplate reader being e.g. the microplate reader used in the present examples.

In a preferred embodiment of any of the above aspects, the format of the method is a NanoBRET protein:protein interaction format including the respective components as outlined above and a suitable detection as well as quantification device. Such assays, components and suitable devices are well known to the skilled person except for the RBX1/NEDD8 fusion protein used in the method of the claimed invention.

In another preferred embodiment of any of the above aspects, RBX1 is fused to a protein as mentioned in any of the above aspect.

### BRIEF DESCRIPTION OF THE FIGURES

**Figure 1A** schematically shows the setup of a method of assessing the induction of proximity by a molecular glue, where the target protein (Target) and the substrate receptor (SR) of a CRL complex are tagged, e.g. with the tags as indicated.
**Figure 1B** schematically shows the setup of a method of the present invention of assessing the induction of proximity by a molecular glue (here for an RBX1 fusion protein), where the target protein (tagged with Nluc) and RBX1 of a CRL complex are tagged, e.g. with the tags as indicated. The location of NEDD8 in the complex is also indicated in the present figure.
**Figure 2** shows the input and the results of the immunoprecipitation experiment of example 1 of the present application. See example 1 for further details.
**Figure 3** shows the results of the RBX1-proximity assay of example 2 of the present application. See example 2 for further details.
**Figure 4** shows the results of the RBX1 -proximity assay of example 3 of the present application when expressing BRD4-BD. See example 3 for further details.
**Figure 5** shows the results of the RBX1 -proximity assay of example 3 of the present application when expressing full-length BRD4. See example 3 for further details.
**Figure 6** shows the results of the RBX1-proximity assay of example 4 of the present application. See example 4 for further details.
**Figure 7** shows the results of the RBX1-proximity assay of example 5 of the present application. See example 5 for further details.

### DETAILED DESCRIPTION OF THE INVENTION

### Definitions

As used herein, the singular form of "a" or "an" also includes the corresponding plural unless the context clearly dictates otherwise.

The term "about" in the context of the present disclosure denotes an interval of accuracy that a person skilled in the art will understand to still ensure the technical effect of the feature in question. The term typically indicates a deviation from the indicated numerical value of ±10% and preferably ±5%.

It needs to be understood that the term "comprising" is not limiting. For the purposes of the present disclosure, the term "consisting of' is considered to be a preferred embodiment of the term "comprising". If hereinafter a group is defined to comprise at least a certain number of embodiments, this is also meant to encompass a group which preferably consists of these embodiments only.

The term "induces proximity" or "induction of proximity" or the like as used herein is used in the meaning of Stanton et al., Science 2018 March 09; 359(6380). As mentioned in the abstract thereof, the induction of proximity, or the physical closeness of molecules, is a pervasive regulatory mechanism in biology, examples being the recruitment of proteins through posttranslational modifications such as phosphorylation, methylation and acetylation. It is further mentioned therein that chemically induced proximity allows for precise temporal control of *inter alia* degradation, which is of particular relevance for the present invention. Stanton et al., *supra,* disclose more background in this respect in the chapter "Degrading or inactivating pathogenic proteins".

The term "target protein" as used herein refers to a protein comprised in a cell that ultimately is to be degraded by inducing the tagging of the protein via ubiquitin for proteasomal degradation. An important step on the route to this "artificially induced" degradation is the step of chemically inducing proximity between the target protein and a CRL complex. As noted in the background section, target proteins of particular interest are proteins implicated in diseases, which have proven to be difficult targets for the more classical approaches of modulating their activity (e.g. when it is rather difficult to screen for and identify inhibitory compounds) or which have additional functions that exceed their catalytic activity (e.g. scaffolding functions). Typically, such proteins are overexpressed and/or overactivated and known to be implicated in a disease of the human, such as oncogenes expressed in/driving cancer.

The term "Cullin-RING E3 ubiquitin ligase (CRL) complex" as used herein refers to the CRL complex known to the skilled person, e.g. as described in Bulatov and Ciulli, *supra.* As noted therein, CRL complexes are the largest family of E3 ubiquitin ligases and have a modular setup.

The term "RBX1" as used herein refers to the protein RBX1 in homo sapiens, alternative names being "E3 ubiquitin-protein transferase RBX1", "Protein ZYP", "RING finger protein 75", "RING-box protein (Rbx1)" and "Regulator of cullins 1 (ROC1)". RBX1 has the amino acid sequence of SEQ ID NO:17. The primary accession number in UniProt is P62877.

The term "NEDD8" as used herein refers to the protein NEDD8 in homo sapiens, alternative names being "Neddylin", "Neural precursor cell expressed developmentally down-regulated protein 8" and "Ubiquitin-like protein Nedd8". NEDD8 has the amino acid sequence of SEQ ID NO:44. The primary accession number in UniProt is Q15843.

The term "induces complex formation" or "induction of complex formation" or the like as used herein are synonymous terms for an "induction of proximity" as defined above, wherein the focus is on a complex formation between a target protein and a CRL complex. The same applies for the term "induces recruitment" or "induction of recruitment" or the like as used herein, which focuses on the recruitment of a target protein to a CRL complex (or *vice versa*, i.e. the recruitment of a CRL complex to a target protein).

The term "compound" as used herein refers to any molecule potentially capable of chemically inducing proximity between a target protein and a CRL complex. Liu and Ciulli, *supra,* provides in Figure 1 some generally suitable molecules, which includes a small molecule, a PROTAC, a nucleic acid (RNA or DNA) fused to a small molecule or peptide/protein, an aptamer, a fused antibody and a macrocycle. The term "compound" as used herein also includes a peptide. Such a peptide may be added to the cell in that it is added from the outside of the cell or such a peptide may be added to the cell in that it is expressed inside the cell (in the latter case, it can be preferred that the expression of the peptide is inducible inside the cell).

The term "peptide" as used herein refers to a compound comprised of amino acid residues covalently linked by peptide bonds and is typically used interchangeably with the terms "protein" and "polypeptide". A peptide must contain at least two amino acids, and no limitation is placed on the maximum number of amino acids that can comprise a peptide's sequence. Peptides include any peptide comprising two or more amino acids joined to each other by peptide bonds. As used herein, the term refers to both short chains, which also commonly are referred to in the art as peptides, oligopeptides and oligomers, for example, and to longer chains, which generally are referred to in the art as proteins, of which there are many types. "Peptides" include, for example, biologically active fragments, substantially homologous peptides, oligopeptides, homodimers, heterodimers, variants of peptides, modified peptides, derivatives, analogs, fusion proteins, among others. The peptides include natural peptides, recombinant peptides, synthetic peptides, or a combination thereof.

The term "small molecule" as used herein refers to a small organic compound having a low molecular weight. The term does not include a "PROTAC" as defined below. The term does include a "molecular glue" as defined below. A small molecule may be a synthetic compound not known to occur in nature or a naturally-occurring compound isolated from or known to occur in natural sources, such as e.g. cells, plants, fungi, animals and the like. A small molecule in the context of the present invention preferably has a molecular weight of less than 5000 Dalton, more preferably of less than 4000 Dalton, more preferably less than 3000 Dalton, more preferably less than 2000 Dalton or even more preferably less than 1000 Dalton. In a particularly preferred embodiment a small molecule in the context of the present invention has a molecular weight of less than 800 Dalton. In another preferred embodiment, a small molecule in the context of the present invention has a molecular weight of 50 to 3000 Dalton, preferably of 100 to 2000 Dalton, more preferably of 100 to 1500 Dalton and even more preferably of 100 to 1000 Dalton. Most preferably, a small molecule in the context of the present invention has a molecular weight of 100 to 800 Dalton. It can be preferred that a small molecule in the context of the present invention meets the "Rule of Five" as set out below and is thus orally active (i.e. has a good oral bioavailability). These rules are as follows: (i) the small molecule has no more than five hydrogen bond donors (e.g. nitrogen or oxygen atoms with one or more hydrogen atoms); (ii) the small molecule has not more than ten hydrogen bond acceptors (e.g. nitrogen or oxygen atoms); (iii) the small molecule has a molecular mass of less than 500 Dalton; (iv) the small molecule has an octanol-water partition coefficient log P not greater than 5.

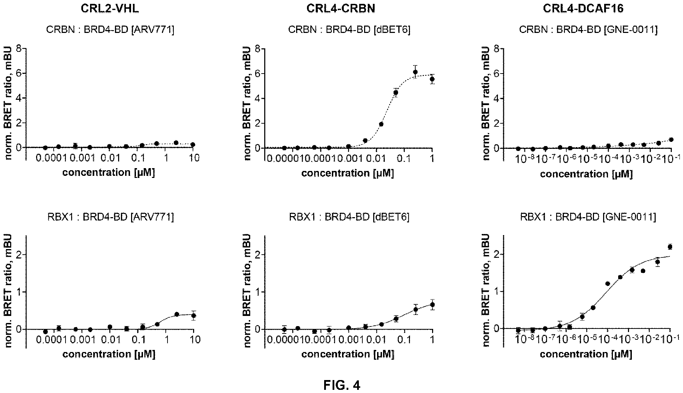

The term "PROTAC" as used herein refers to a bifunctional, more specifically a heterobifunctional, molecule consisting of two binding modules that are connected by a linker: one binding module binds to the target protein and one binding module binds to an E3 ligase component. The term is used herein in its common understanding in the field, e.g. as expressed in Liu and Ciulli, *supra.* As mentioned therein, there is a high number of PROTACs presently undergoing clinical trials, wherein the targets and the respective PROTACs are indicated in Figure 4 of Liu and Ciulli, *supra,* examples being DT2216, CFT8634 and ASP3082.

The term "molecular glue" as used herein is used in its common understanding in the field, as e.g. expressed in Holdgate et al., SLAS Discovery 29 (2024) 100136 and Liu and Ciulli, *supra.* As mentioned therein, a molecular glue can aim to stabilize the interaction between two proteins, but a molecular glue can also induce other interactions that e.g. stabilize interactions to increase activity or to inhibit binding to a natural effector. Further, a molecular glue can be characterized as monomeric molecule (in contrast e.g. to a bifunctional PROTAC) and the best-known examples of molecular glues are thalidomide including its derivatives pomalidomide and lenalidomide, as well as indisulam and E7820.

### Examples

### EXAMPLE 1: Expression constructs and immunoprecipitation assay to test for the expression and complex-formation of the proteins

The plasmids shown in **Table Ex-1** expressing the tagged proteins as indicated under the control of a CMV promoter were designed and synthesized or commercially obtained:

**Table Ex-1**

| **Plasmid (sequence of SEQ ID as indicated)** | **Source** | **Protein** | **Tags** | **Tagged terminus** | **Linker** |
|---|---|---|---|---|---|
| pCMV_RBX1_1xG4S _Halo_N (SEQ ID NO:1) | Synthesized by Twist | RBX1 | HaloTag, 2xHA | N | 1xG4S |
| pCMV_RBX1_1xG4S _Halo_C (SEQ ID NO:2) | Synthesized by Twist | RBX1 | HaloTag, 2xHA | C | 1xG4S |
| pCMV_RBX1_3xG4S _Halo_N (SEQ ID NO:3) | Synthesized by Twist | RBX1 | HaloTag, 2xHA | N | 3xG4S |
| pCMV_RBX1_3xG4S _Halo_C (SEQ ID NO:4) | Synthesized by Twist | RBX1 | HaloTag, 2xHA | C | 3xG4S |
| pCMV_RBX1_1xG4S _Nluc_N (SEQ ID NO:5) | Synthesized by Twist | RBX1 | Nluc, 2xHA | N | 1xG4S |
| pCMV_RBX1_1xG4S _Nluc_C (SEQ ID NO:6) | Synthesized by Twist | RBX1 | Nluc, 2xHA | C | 1xG4S |
| pCMV_RBX1_3xG4S _Nluc_N (SEQ ID NO:7) | Synthesized by Twist | RBX1 | Nluc, 2xHA | N | 3xG4S |
| pCMV_RBX1_3xG4S _Nluc_C (SEQ ID NO:8) | Synthesized by Twist | RBX1 | Nluc, 2xHA | C | 3xG4S |
| pCMV_BRD4_BD_H alo_N (SEQ ID NO:9) | Synthesized by Twist | BRD4-BD | HaloTag, 2xHA | N | 1xG4S |
| pCMV_BRD4_BD_NI uc_N (SEQ ID NO:10) | Synthesized by Twist | BRD4-BD | Nluc, 2xHA | N | 1xG4S |
| PCMV_BRD4_FL_NI uc_N (SEQ ID NO:11) | Promega Cat #N1691 | BRD4-FL | Nluc | N | n.a. |
| HaloTag-CRBN fusion vector (SEQ ID NO:12) | Promega Cat #N2691 | CRBN | HaloTag | N | n.a. |

As indicated, two different linkers between the proteins and the tags were used, namely either the amino acid sequence GGGGS (referred to as "1xG4S", SEQ ID NO:13) or GGGGSGGGGSGGGGS (referred to as "3xG4S", SEQ ID NO:14).

The tags "HaloTag" with the protein sequence of SEQ ID NO:15 and "Nluc" with the protein sequence of SEQ ID NO:16 were used.

When RBX1 (with the protein sequence of SEQ ID NO: 17) was tagged N-terminally with either HaloTag or Nluc, the construct always included a 2xHA-tag (with the protein sequence of SEQ ID NO:18) as the very first tag at the N-terminus, and the starting methionine (M) of the RBX1 sequence was omitted. When RXB1 was tagged C-terminally with either HaloTag or Nluc, the construct always included a 2xHA tag as the very last tag at the C-terminus, and the starting methionine (M) of the HaloTag or Nluc was omitted.

BRD4 was N-terminally tagged either as a partial sequence of the first bromodomain (BRD4-BD with the protein sequence of SEQ ID NO:19) or as the full-length sequence (BRD4-FL with the protein sequence of SEQ ID NO:20). BRD4-BD was tagged N-terminally with either HaloTag or Nluc, and the construct included a 2xHA-tag as the very first tag at the N-terminus. CRBN with the protein sequence of SEQ ID NO:21 was used. N-terminally tagged full length BRD4 with Nluc and N-terminally Halo-tagged CRBN were purchased from Promega (#N1691 and #N2691, respectively).

### Cullin ring ubiquitin E3 ligase (CRL) complex immunoprecipitation

A CRL complex immunoprecipitation experiment was performed to test if (i) HA-HaloTag-linker-RBX1, (ii) HA-NanoLuc-linker-RBX1, (iii) RBX1-linker-HaloTag-HA and (iv) RBX1-linker-NanoLuc-HA fusion proteins are transiently expressed in HEK293T cells and able to interact with the further components of the CRL complex, such as Cullins. To this aim, pulldown experiments of the different versions of RBX1, using the HA-tag, were performed, followed by Western Blot analysis to identify the interacting proteins. Pulldowns were performed for both N- and C-terminally tagged versions of fusion proteins (i) to (iv) above, connected either by the 1xG4S or the 3xG4S linker.

In the morning of the first day, 5×10⁶ HEK293T WT cells were seeded in DMEM supplemented with 10% FBS and 1% Pen/Strep (DMEM+/+) per 10 cm plate. Cells were allowed to attach and recover for 4-6 hours at 37°C, 5% CO₂. Cells were transfected with 1.5 µg of either a HaloTag-RBX1 fusion construct (pCMV_RBX1_1xG4S_Halo_N, pCMV_RBX1_1xG4S_Halo_C, pCMV_RBX1_3xG4S_Halo_N, or pCMV_RBX1_3xG4S_Halo_C), a Nluc-RBX1 fusion construct (pCMV_RBX1_1xG4S_Nluc_N, pCMV_RBX1_1xG4S_Nluc_C, pCMV_RBX1_3xG4S_Nluc_N, or pCMV_RBX1_3xG4S_Nluc_C) or an empty control plasmid (pcDNA3.1(-)) using Polyethylenimine (PEl, Polysciences, Cat# 24765-1) as transfection reagent (1:5 ratio of µg DNA for µl of 1 mg/ml PEI). Cells were incubated for 48 hours at 37°C, 5% CO₂, then collected from the 10 cm plates and resuspended in 1.5 ml of NP-40 lysis buffer (50mM Tris pH 7.5, 225mM KCI, 5% Glycerol, 0.5 % NP-40, 1.5mM MgCl₂, 1X Protease inhibitor (ThermoFisher, Cat #78429), 0.25 U/ml Benzonase Nuclease (Sigma, Cat #E1014)). Samples were incubated 30 min on ice for lysis and afterwards spun for 30 min at maximum speed (14000 rpm). The supernatants were collected as lysates and 5% of each lysate was stored in 1xLaemmli buffer at -20°C as input samples for western blot analysis. Per sample, 25 µl Anti-HA magnetic beads (Thermo Fisher, Cat #88836) were washed twice in PBS-T (PBS + 0.05% Tween-20), resuspended in 25 µl of NP-40 lysis buffer and added to cell lysate. Lysates were incubated with beads on a rotational shaker at 4°C for 4 hours in the cold room. Beads were washed five times in NP-40 buffer. After the last washing step, wash buffer was removed and 100 µl of 1x Laemmli buffer was added, and beads were boiled at 95°C for 3 min. Supernatant was collected as immunoprecipitation sample (IP) and stored at -20°C. For western blot analysis, 20% of IP and 0.1% of input samples were boiled for 5 min at 95°C, loaded on a Bolt Bis-Tris Plus 4-12% gel (ThermoFisher, Cat #NW04125BOX) and ran using Bolt MES SDS running buffer (ThermoFisher, Cat #B000202) followed by transfer to a 0.45 µm nitrocellulose western blot membrane (Sigma, Cat #GE10600002) using Bolt transfer buffer (ThermoFisher, Cat #BT0006). Membrane was blocked in 5% milk in TBS-T (20 mM Tris, 150 mM NaCl, 0.1% Tween-20, pH 7.6), briefly washed in TBS-T, and incubated in primary antibody at 4°C on a rolling shaker overnight. Membrane was washed with TBS-T and incubated with secondary antibody in 5% milk in TBS-T for 1 h at room temperature. Membrane was washed with TBS-T, briefly incubated with chemiluminescent substrate (Thermo Fisher, Cat# 32106) and developed using an imager (Odyssey XF, LI-COR).

The following primary and secondary ABs were used:
- Anti-HA rabbit mAb: Cell signaling Cat# 3724
- Anti-CUL4B rabbit pAB: Eurogentec Cat# 12916-1-AP
- Anti-DDB1 rabbit pAB: Cell signaling Cat# 5428S
- Anti-CRBN rabbit mAB: Cell signaling Cat# 71810S
- Anti-rabbit IgG Peroxidase AffiniPure Secondary pAB: Jackson ImmunoResearch, Cat# 111-035-003; and
- Anti-mouse IgG Peroxidase Secondary pAB: Jackson ImmunoResearch, Cat#115-035-003

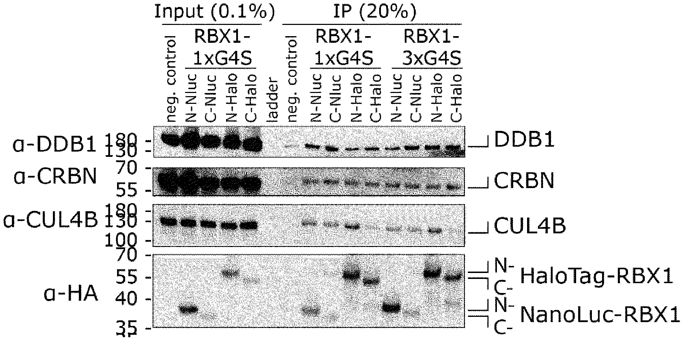

The results of the pull-down are shown in Figure 2. On the left side, the input is shown (for one set of linkers, namely the 1xG4S linkers). On the right side, the IP-samples of the different pull-downs are shown. As can be derived from these samples, the expected components of the CRL complex were pulled-down together with RBX1, namely DDB1, CRBN and CUL4B. Accordingly, N- as well as the C-terminally tagged RBX1 was still incorporated into the CRL complex. It seems that the C-terminal tagging of RBX1 results in slightly lower protein levels of the corresponding fusion proteins for both tags (i.e. HaloTag and Nluc). Further, it seems that the highest level of CUL4B was pulled down in the IP using the N-terminally HaloTag-tagged RBX1, and that this result was independent from whether the shorter (1xG4S) or the longer (3xG4S) linker was used.

In summary, the present example shows that N- or C-terminally tagged RBX1 still incorporates into the CRL complex, i.e. it retains its endogenous interactions and function.

### EXAMPLE 2: Proof of concept of an RBX1 proximity assay

The RBX1-proximity assay was performed with RKO WT cells transfected with a mixture of (a) pCMV_BRD4_BD_Nluc_N and (b)(i) pCMV_RBX1_1xG4S_Halo_N or (b)(ii) pCMV_RBX1_3xG4S_Halo_N. Alternatively, RKO WT cells were transfected with a mixture of (c) pCMV_BRD4_BD_Halo_N and (d)(i) pCMV_RBX1_1xG4S_Nluc_N or (d)(ii) pCMV RBX1 3xG4S_Nluc N.

In the morning of the first day, 1×10⁶ RKO WT cells were seeded in 2 ml of DMEM supplemented with 10% FBS, 1% Pen/Strep and 1 % Sodium pyruvate (DMEM+/+/+) per well of a 6-well plate. Cells were allowed to attach and recover for 4-6 hours at 37°C, 5% CO₂. The cells were then transfected with a mixture of 1.5 µg HaloTag-plasmid (either CMV_RBX1_1xG4S_Halo_N, CMV_RBX1_3xG4S_Halo_N or pCMV_BRD4_BD_Halo_N) in combination with 15 ng of Nluc-plasmid (either CMV_RBX1_1xG4S_Nluc_N, CMV_RBX1_3xG4S_Nluc_N or pCMV_BRD4_BD_Nluc_N) using Polyethylenimine (PEl, Polysciences, Cat# 24765-1) as transfection reagent (1:5 ratio of µg DNA for µl of 1 mg/ml PEI). Cells were incubated for 20-24 hours at 37°C, 5% CO₂. In the afternoon of the second day, cells were collected and resuspended in assay medium (Opti-MEM I Reduced Serum Media, no phenol red (Life Technologies Cat# 11058-021) + 4% FBS, + 1% pen/strep) at a concentration of 0.22×10⁶ cells/ml. 100 nM HaloTag 618 Ligand (Promega, # N1662) was added to cell suspension before seeding 90 µl into white Opaque 96-well assay plates (Revvity, Cat # 6055680) (20.000 cells/well). Cells were incubated overnight at 37°C, 5% CO₂. In the morning of the third day, cells were treated with 0.01% DMSO (control), 1 µM ARV771 or 1 µM) dBET6 for 60 min. For this, 10x concentrated compound stocks were prepared in assay medium and 10 µl were added to cells in duplicates. Assay plates were spun down quickly at 250 × g and incubated for 60 min at 37°C, 5% CO₂. Before the readout, 25 µl of 5x stock solution of NanoBRET Nano-Glo substrate (Promega, # N1662) was added to the assay plates. The plates were quickly spun down at 250 × g prior to measurement. NanoBRET measurement was performed using the Envision 2105 (Revvity). For this, donor emission at 460 nm (emission filter NanoBRET Blue at 460/75, Perkin Elmer# 2100-5950) and acceptor emission at 647 nm (emission filter BRET Deep Red at 647/75, Perkin Elmer # 2100-5970) were measured with 1s integration time.

The normalized NanoBRET ratio was calculated as follows:
1. For each sample, the acceptor emission value (618nm) was divided by the donor emission value (460nm) and multiplied by 1000 to generate the raw NanoBRET ratio values (mBU).
2. The average NanoBRET ratio for the experimental samples was determined.
3. The average NanoBRET ratio of DMSO samples was subtracted from the NanoBRET ratio of compound-treated samples to generate the normalized NanoBRET ratios (norm. mBU).

In the present assay, the bromodomain of BRD4 is expressed in the form of a fusion protein, where either Nluc or HaloTag is fused to the N-terminus of the bromodomain.

ARV771 is an established PROTAC that on the one hand binds to the bromodomain of BRD4 and on the other hand binds to VHL of the CRL2-VHL complex (Raina, K. et al. PROTAC-induced BET protein degradation as a therapy for castration-resistant prostate cancer. Proc. Natl Acad. Sci. USA 113, 7124-7129 (2016)). RBX1 is also part of this CRL complex and expressed in the present setup either as HaloTag-RBX1 or as Nluc-RBX1.

dBET6 is an established PROTAC that on the one hand binds to the bromodomain of BRD4 and on the other hand binds to CRBN of the CRL4-CRBN complex (Winter, G. E. et al. BET bromodomain proteins function as master transcription elongation factors independent of CDK9 recruitment. Mol. Cell 67, 5-18 (2017)). RBX1 is also part of this CRL complex and expressed in the present setup either as HaloTag-RBX1 or as Nluc-RBX1.

When either co-expressing (a) pCMV_BRD4_BD_Nluc_N and (b)(i) pCMV_RBX1_1xG4S_Halo_N or (b)(ii) pCMV_RBX1_3xG4S_Halo_N; or (c) pCMV_BRD4_BD_Halo_N and (d)(i) pCMV_RBX1_1xG4S_Nluc_N or (d)(ii) pCMV_RBX1_3xG4S_Nluc_N, CRL complex formation is expected after the addition of ARV771 (the CRL then being the CRL2-VHL complex) or dBET6 (the CRL then being the CRL4-CRBN complex). It is noted that both complexes, the CRL2-VHL complex and the CRL4-CRBN complex, contain RBX1 as a component. Accordingly, irrespective of the specific complex, the normalized NanoBRET ratio should provide a positive result, i.e. it should indicate that there is proximity between (the tagged) RBX1 and the (tagged) bromodomain of BRD4.

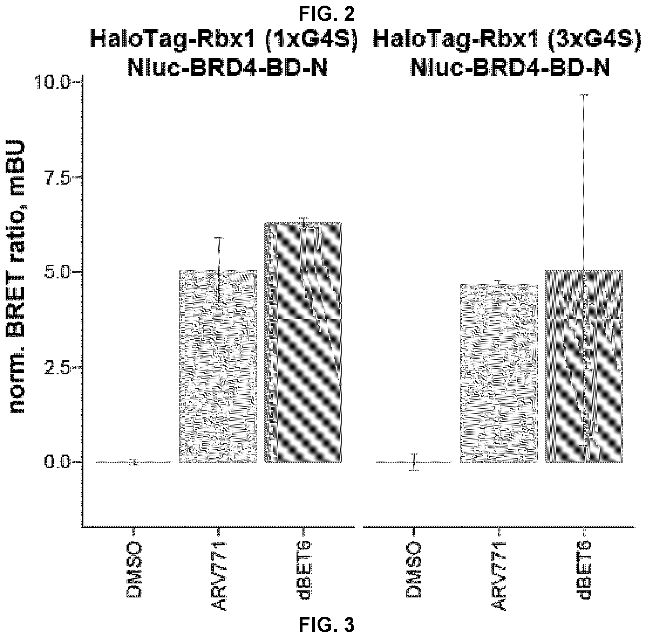

Figure 3 shows the results for HaloTag-tagged RBX1, either with the 1XG4S-linker or the 3xG4S-linker, co-expressed with the Nluc-BRD4-bromodomain. Clearly, the addition of either ARV771 or dBET6 resulted in a positive normalized BRET ratio of about 5 for ARV771 and about 6 for dBET6, wherein the length of the linker had no substantial impact. The results for the inverted setup for Nluc-RBX1, either with the 1XG4S-linker or the 3xG4S-linker, co-expressed with the HaloTag-BRD4-bromodomain indicated a positive normalized BRET ratio for the shorter linker, albeit to a lower level for both ARV771 and dBET6 (of about 2, data not shown).

In summary, the present example shows that HaloTag-tagged RBX1, which is present in the CRL2-VHL complex and the CRL4-CRBN complex, is, as indicated by a positive BRET ratio, brought into proximity to the bromodomain of BRD4 if either ARV771 is added to induce a complex between BRD4 and the CRL2-VHL complex or dBET6 is added to induce a complex between BRD4 and the CRL4-CRBN complex. The length of the linker in the RBX1-fusion proteins does not play a substantial role.

### EXAMPLE 3: RBX1-tagging is widely applicable

As indicated in Figure 1A, the substrate receptor is tagged in the traditionally used protocols. Such a substrate receptor can e.g. be VHL (in the CRL2-VHL complex), CRBN (in the CRL4-CRBN complex) or DCAF16 (in the CRL4-DCAF16 complex). The aim of the present example was to show that the tagging of a substrate receptor allows for a readout on the level of a specific CRL complex, where this particular substrate receptor is involved, whereas the tagging of RBX1 allows for a wider readout, namely on a level of all CRL complexes, where RBX1 is involved.

The proximity assay was performed with HEK293T cells transfected with a mixture of (a) pCMV_BRD4_BD_Nluc_N and (b)(i) pCMV_CRBN_Halo_N or (b)(ii) pCMV_RBX1_1xG4S_Halo_N. Compared to Example 2 described above, not only ARV771 or dBET6 but alternatively GNE-0011 was added to the cells. The transfection of cells was carried out as described above in Example 2. In the afternoon of the second day after transfection, cell medium was exchanged with 2 ml assay medium (Opti-MEM I Reduced Serum Media, no phenol red (Life Technologies Cat# 11058-021) + 4% FBS, + 1% pen/strep) containing 100 nM HaloTag 618 Ligand (Promega, # N1662). Cells were incubated overnight at 37°C, 5% CO₂. In the morning of the third day, cells were collected and resuspended in assay medium (Opti-MEM I Reduced Serum Media, no phenol red (Life Technologies Cat# 11058-021) + 4% FBS, + 1% pen/strep) at a concentration of 0.16 M cells/ml, before seeding 50 µl into white Opaque 384-well assay plates (Fisher Scientific, Cat# 10199622) (8.000 cells/well) pre-spotted with compounds using the Echo 650 liquid handler system (Beckman Coulter). Assay plates were spun down quickly at 250 × g and incubated for 60 min at 37°C, 5% CO₂. Before the readout, 10 µl of 6x stock solution of NanoBRET Nano-Glo substrate (Promega, # N1662) was added to the assay plates. The plates were quickly spun down at 250 × g prior to measurement.

NanoBRET measurement and the calculation of normalized NanoBRET was carried out as described above in Example 2.

In all of the above co-expression scenarios, the following situations arise depending on the addition of either ARV771, dBET6 or GNE-0011: ARV771 will induce a ternary complex between the BRD4 bromodomain and the CRL2-VHL complex; dBET6 will induce a ternary complex between the BRD4 bromodomain and the CRL4-CRBN complex; and GNE-0011 will induce a ternary complex between the BRD4 bromodomain and the CRL4-DCAF16 complex (Hsia, O., Hinterndorfer, M., Cowan, A.D. et al. Targeted protein degradation via intramolecular bivalent glues. Nature 627, 204-211 (2024). https://doi.org/10.1038/s41586-024-07089-6). Accordingly, in all three situations, there should be a positive signal for complex formation.

In the scenario, where the CRBN is tagged (see (b)(i) above), it is expected that the complex formation is, however, only detectable for the CRL4-CRBN complex, because CRBN is not part of the CRL2-VHL and the CRL4-DCAF16 complex, respectively. In line with this, a complex formation is only detectable via the normalized BRET ratio in the assay, where dBET6 had been added (see row "CRL4-CRBN", upper graph in Figure 4). Although complexes also form after addition of ARV771 (CRL2-VHL complex, see above) and GNE-0011 (CRL4-DCAF16, see above), these are not detected if the substrate receptor CRBN is tagged (see rows "CRL2-VHL" and "CRL4-DCAF16", upper graphs in Figure 4).

The picture is different if RBX1 is tagged (see (b)(ii) above): Here, complex formation should be detectable for all three scenarios, where complexes are induced, i.e. after addition of either ARV771, dBET6 or GNE-0011, because RBX1 is part of the CRL2-VHL complex, the CRL4-CRBN-complex and the CRL4-DCAF16 complex. Indeed, the results show complex formation in all three scenarios, see the lower graphs in all three rows in Figure 4.

The experiments described above were repeated in an almost identical setup. Thus, in the setup described in the following, not only the bromodomain of BRD4 was expressed but the full length BRD4 protein. The aim of these additional experiments was to verify whether recruitment of larger proteins could also be detected by the assay, without the distance between the Nluc and the HaloTag exceeding the range conductive for energy transfer.

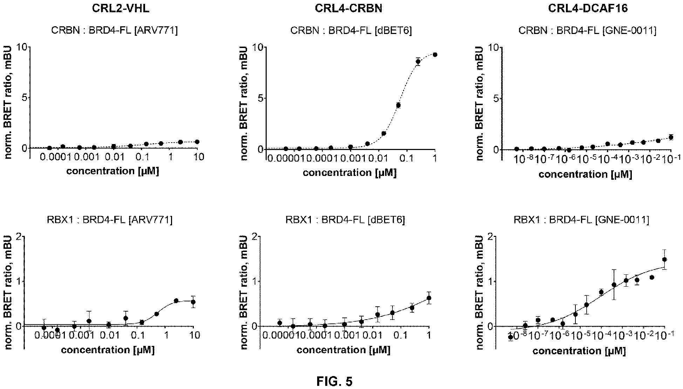

The results obtained with the full length BRD4 protein are nicely in line with the results obtained for the bromodomain of BRD4 as discussed above: If CRBN is tagged, the complex formation is only detected if dBET6 is added but not upon ARV771 or GNE-0011 addition (see the upper graphs of all three rows of Figure 5). In contrast thereto, when RBX1 is tagged, complex formation is nicely detected in all three scenarios (see the lower graphs of all three rows of Figure 5).

In summary, the present example shows that the tagging of RBX1 has a wider applicability in terms of CRL complexes compared to a tagged substrate receptor, as carried out in the traditionally used protocols.

### EXAMPLE 4: Validation of the RBX1-tagged assay with other targets

The aim of the present examples was to validate the RBX1-proximity assay using other targets. In the present example, the RBX1-proximity assay was performed in HEK293T cell lines, each cell line stably expressing either Nluc-tagged CCNK, Nluc-tagged CDK2 or Nluc-tagged P300. Each cell line was transfected with pCMV_RBX1_1xG4S_Halo_N as outlined above in Example 2 to also express the HaloTag-tagged RBX1 protein.

The cell lines were obtained as follows, wherein further details can be derived from **Table Ex-2:**
To obtain HEK293T cells stably expressing Nluc-tagged CCNK (the protein sequence of CCNK is shown in SEQ ID NO:22), HEK293T cells were engineered using the PITCh system (sgRNA sequence: AAGCCTACTTCAATAAATGA (SEQ ID NO:23)) as previously described in Sakuma, T. et al. MMEJ-assisted gene knock-in using TALENs and CRISPR-Cas9 with the PITCh systems. Nat Protoc 11, 118-133 (2016). https://doi.org/10.1038/nprot.2015.140, resulting in heterozygous N-terminal tagging of the endogenous CCNK protein with a 2xHA-tag and Nluc, separated from each other by a GSG sequence, followed by a 3xG4S linker and the sequence of CCNK.

To obtain HEK293T cells stably expressing CDK2 (the protein sequence of CDK2 is shown in SEQ ID NO:24), HEK293T cells were engineered using lentiviral integration according to a standard protocol resulting in expression (under the control of the lentiviral overexpression promoter hPGK) of additional CDK2 protein with Nluc and an additional 2xHA-tag at the N or C-terminus. The 2xG4S-attB1 linker sequence (SEQ ID NO:25) was used as linker between the protein and the N-terminal tags and the attB2-2xG4S sequence (SEQ ID NO:26) was used as linker between the protein and the C-terminal tags. In the present example, the cell line stably expressing the N-terminally tagged version of CDK2 was used.

To obtain HEK293T cells stably expressing P300 core domain (the protein sequence of the p300 core domain is shown in SEQ ID NO:27), HEK293T cells were engineered using lentiviral integration according to a standard protocol resulting in expression (under the control of the lentiviral overexpression promoter hPGK) of additional P300-core protein tagged with Nluc and an additional 2xHA-tag at the C-terminus. The 1xG4S sequence was used as linker between the protein and the C-terminal tags.

**Table Ex-2** summarizes the details of the cell lines that were used in Example 4.

**Table Ex-2**

| **Cell line** | **Promoter** | **Protein** | **Tagged terminus** | **Tag** | **Linker** |
|---|---|---|---|---|---|
| CCNK_Nluc_N | endogenous | CCNK | N | 2xHA, Nluc | 3xG4S |
| CDK2_Nluc_N | hPGK | CDK2 | N | 2xHA, Nluc | 2xG4S-attB1 |
| CDK2_Nluc_C | hPGK | CDK2 | C | Nluc, 2xHA | attB2-2xG4S |
| P300_Nluc_C | hPGK | P300-core | C | Nluc, 2xHA | 1xG4S |

All experimental steps including the transfection and the determination of the normalized NanoBRET ratio were carried out as described above in Example 2, with the exception that only the RBX1-HaloTag plasmid (pCMV_RBX1_1xG4S_Halo_N) was used for transfection, whereas the respective Nluc-tagged protein was stably expressed in the cell lines as indicated above. The cells were treated in duplicates with either DMSO or their respective degrader compound: (i) CR8 for CCNK, see Stabicki M, et al. The CDK inhibitor CR8 acts as a molecular glue degrader that depletes cyclin K. Nature. 2020 Sep;585(7824):293-297. doi: 10.1038/s41586-020-2374-x. Epub 2020 Jun 3. PMID: 32494016; PMCID: PMC7486275 for a 60 min treatment; (ii) PROTAC CDK2/9 Degrader-1 for CDK2, see Zhou F, et al. Development of selective mono or dual PROTAC degrader probe of CDK isoforms. EurJ Med Chem. 2020 Feb 1;187:111952. doi: 10.1016/j.ejmech.2019.111952. Epub 2019 Dec 6. PMID: 31846828 (MedChemExpress, Cas# 2408641-24-5) for a 120 min treatment; and (iii) and PROTAC dCBP1 for P300 (Vannam R, et al. Targeted degradation of the enhancer lysine acetyltransferases CBP and p300. Cell Chem Biol. 2021 Apr 15;28(4):503-514.e12. doi: 10.1016/j.chembiol.2020.12.004. Epub 2021 Jan 4. PMID: 33400925 (MedChemExpress, Cas# 2484739-25-3) for a 120 min treatment.

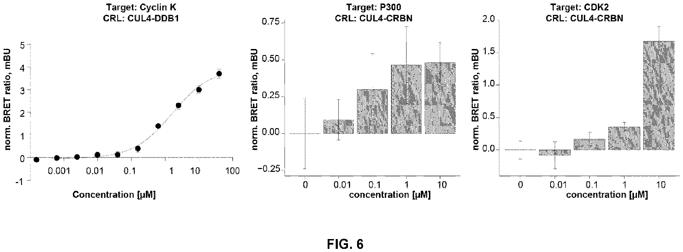

The results shown in Figure 6 nicely show that complex formation is detected in all three scenarios if RBX1 is tagged, see the left graph, where CR8 as degrader was added in the concentrations as indicated, the middle graph, where PROTAC CDK2/9 Degrader-1 was added in the concentrations as indicated, and the right graph, where PROTAC dCBP1 for P300 was added in the concentrations as indicated: for all three scenarios, a concentration dependent complex formation can be detected.

In summary, the present example shows that the tagging of RBX1 works for a variety of targets.

### EXAMPLE 5: Complex formation is detected for CDK2 irrespective of whether the tag is fused N- or C-terminally

The aim of the present examples was to determine whether the assay is functional independently of the location (i.e. N- or C-terminally) of the Nluc-tag on the protein to be degraded.

The two HEK293T cell lines of example 4 stably expressing an N-terminal Nluc CDK2 and a C-terminal Nluc CDK2 fusion protein, respectively, were used. Each cell line was transfected with pCMV_RBX1_1xG4S_Halo_N as outlined above in Example 2 to also express the HaloTag-tagged RBX1 protein.

All experimental steps including the transfection and the determination of the normalized NanoBRET ratio were carried out as described above in Example 2, with the exception that only the RBX1-HaloTag plasmid (pCMV_RBX1_1xG4S_Halo_N) was used for transfection, whereas the N-terminally and C-terminally Nluc-tagged CDK2, respectively, was stably expressed in the cell lines as indicated above. The cells were treated in duplicates with either DMSO or with PROTAC CDK2/9 Degrader-1 for CDK2 for a 120 min treatment.

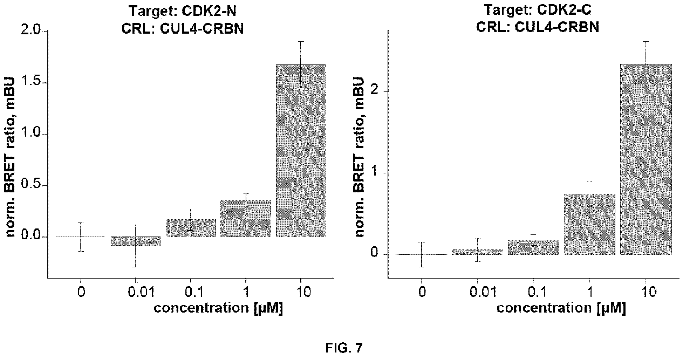

The results shown in Figure 7 nicely show that complex formation is detected in both setups, i.e. independently from whether CDK2 is N- or C-terminally tagged with Nluc, see the graphs, where PROTAC CDK2/9 Degrader-1 was added in the concentrations as indicated: a concentration dependent complex formation can be detected for both, the N- and the C-terminally tagged CDK2.

## Claims

1. An *in vitro* method of determining whether a compound induces proximity between (i) a target protein and (ii) a Cullin-RING E3 ubiquitin ligase complex, the method comprising the following steps:
a. Providing a cell comprising
i. a target protein fused to a protein being an energy donor and RBX1 or NEDD8 fused to a protein being an energy acceptor; or
ii. a target protein fused to a protein being an energy acceptor and RBX1 or NEDD8 fused to a protein being an energy donor; or
iii. a target protein fused to a protein being a first protein fragment of a signal-generating protein and RBX1 or NEDD8 fused to a protein being a second fragment of the signal-generating protein, wherein the first fragment and the second fragment are, upon association, capable of forming a functional signal-generating protein;
b. Adding a compound to the cell provided in step a); and
c. Determining for a)i) and a)ii) whether there is energy transfer from the energy donor to the energy acceptor and for a)iii) whether there is a signal of the signal-generating protein in the cell to which the compound has been added in step b);
wherein for a)i) and a)ii) the presence of energy transfer and for a)iii) the presence of a signal indicates that the compound induces proximity between (i) and (ii).

2. An *in vitro* method of screening a plurality of different compounds for the ability of a compound of said plurality of different compounds to induce proximity between (i) a target protein and (ii) a Cullin-RING E3 ubiquitin ligase complex, the method comprising the following steps:
a. Providing a plurality of cells, each cell comprising
i. a target protein fused to a protein being an energy donor and RBX1 or NEDD8 fused to a protein being an energy acceptor; or
ii. a target protein fused to a protein being an energy acceptor and RBX1 or NEDD8 fused to a protein being an energy donor; or
iii. a target protein fused to a protein being a first protein fragment of a signal-generating protein and RBX1 or NEDD8 fused to a protein being a second fragment of the signal-generating protein, wherein the first fragment and the second fragment are, upon association, capable of forming a functional signal-generating protein;
b. Adding each compound of said plurality of different compounds to at least one given cell of the plurality of cells provided in step a) and thereby providing a plurality of given cells, wherein each at least one given cell comprises a specific compound of said plurality of different compounds; and
c. Determining in which of the at least one given cell provided in step b) there is for a)i) and a)ii) energy transfer from the energy donor to the energy acceptor and for a)iii) a signal of the signal-generating protein;
wherein the presence of energy transfer for a)i) and a)ii) and the presence of a signal for a)iii) in an at least one given cell indicates that the specific compound of said plurality of compounds comprised therein has the ability to induce proximity between (i) and (ii).

3. An *in vitro* method of confirming that a compound induces proximity between (i) a target protein and (ii) a Cullin-RING E3 ubiquitin ligase complex, the method comprising the following steps:
a. Providing a cell comprising
i. a target protein fused to a protein being an energy donor and RBX1 or NEDD8 fused to a protein being an energy acceptor; or
ii. a target protein fused to a protein being an energy acceptor and RBX1 or NEDD8 fused to a protein being an energy donor; or
iii. a target protein fused to a protein being a first protein fragment of a signal-generating protein and RBX1 or NEDD8 fused to a protein being a second fragment of the signal-generating protein, wherein the first fragment and the second fragment are, upon association, capable of forming a functional signal-generating protein;
b. Adding a compound to the cell provided in step a); and
c. Determining for a)i) and a)ii) whether there is energy transfer from the energy donor to the energy acceptor and for a)iii) whether there is a signal of the signal-generating protein in the cell to which the compound has been added in step b);
wherein for a)i) and a)ii) the presence of energy transfer and for a)iii) the presence of a signal confirms that the compound induces proximity between (i) and (ii).

4. The *in vitro* method according to any one of claims 1 to 3, wherein for a)i) and a)ii) the determination of energy transfer is carried out after adding a substrate of the protein being an energy donor.

5. The *in vitro* method according to any one of claims 1 to 4, wherein for a)iii) the determination of a signal of the signal-generating protein is carried out after adding a substrate of the signal-generating protein.

6. An *in vitro* method of improving the ability of a compound to induce proximity between (i) a target protein and (ii) a Cullin-RING E3 ubiquitin ligase complex to arrive at an improved compound, the method comprising the following steps:
a. Providing a cell comprising
i. a target protein fused to a protein being an energy donor and RBX1 or NEDD8 fused to a protein being an energy acceptor; or
ii. a target protein fused to a protein being an energy acceptor and RBX1 or NEDD8 fused to a protein being an energy donor; or
iii. a target protein fused to a protein being a first protein fragment of a signal-generating protein and RBX1 or NEDD8 fused to a protein being a second fragment of the signal-generating protein, wherein the first fragment and the second fragment are, upon association, capable of forming a functional signal-generating protein;
b. Adding a compound to the cell provided in step a);
c. Quantifying for a)i) and a)ii) the energy transfer from the energy donor to the energy acceptor and for a)iii) the signal of the signal-generating protein in the cell to which the compound has been added in step b) to arrive at result 1;
d. Modifying the compound added in step b) to arrive at a modified compound;
e. Adding the modified compound of step d) to the cell provided in step a); and
f. Quantifying for a)i) and a)ii) the energy transfer from the energy donor to the energy acceptor and for a)iii) the signal of the signal-generating protein in the cell to which the modified compound has been added in step e) to arrive at result 2;
wherein a ratio of result 2 / result 1 of >1 indicates that the modified compound of step d) is an improved compound.

7. The *in vitro* method according to claim 6, wherein the compound added in step b) and the modified compound added in step e) are added at the same concentration.

8. The *in vitro* method according to claim 6 or 7, wherein in steps c) and f) for a)i) and a)ii) the quantification of energy transfer is carried out after adding a substrate of the protein being an energy donor.

9. The *in vitro* method according to any one of claims 6 to 8, wherein in steps c) and f) for a)iii) the quantification of the signal of the signal-generating protein is carried out after adding a substrate of the signal-generating protein.

10. The *in vitro* method according to any one of claims 1 to 9, wherein the protein being an energy donor is a luciferase.

11. The *in vitro* method according to any one of claims 1 to 10, wherein the protein being an energy acceptor is an enzyme bound to a ligand being a fluorophore, wherein the enzyme is preferably a HaloTag.

12. The *in vitro* method according to any one of claims 1 to 11, wherein the first protein fragment of a signal-generating protein is a fragment of a luciferase and the second protein fragment of a signal-generating protein is a fragment of a luciferase.

13. The *in vitro* method according to any one of claims 1 to 11, wherein the cell comprises i) a target protein fused to a protein being an energy donor and RBX1 or NEDD8 fused to a protein being an energy acceptor.

14. The *in vitro* method according to any one of claims 1 to 13, wherein the protein fused to RBX1 or NEDD8 is fused N-terminally to RBX1 or NEDD8.

15. The *in vitro* method according to any one of claims 1 to 14, wherein the compound is not a PROTAC.
